# EUROPÄISCHE PATENTANMELDUNG

(11) **EP 0 830 878 A1**
(43) Veröffentlichungstag der Anmeldung: **25.03.1998**
(21) Anmeldenummer: 97114811.9
(22) Anmeldetag: 27.08.1997
(51) Int. Cl.: A61N 1/372, A61F 2/00, A61B 5/00

(54) **Vorrichtung zur Rejektionsdiagnostik nach Organtransplantationen**

(30) Priorität: 20.09.1996 DE 19638585
(71) Anmelder: BIOTRONIK Mess- und Therapiegeräte GmbH & Co Ingenieurbüro Berlin, D-12359 Berlin (DE)
(72) Erfinder: Bolz, Armin, Dr., 91054 Buckenhof (DE); Schaldach, Max, Prof. Dr., 91054 Erlangen (DE)
(74) Vertreter: Hübner, Gerd, Dipl.-Phys.

(57) **Zusammenfassung**

Eine Vorrichtung zur Rejektionsdiagnostik nach Organtransplantationen ist versehen mit
- einer extrakorporalen Basisstation (1) mit Hochfrequenz-Sende- (2) und Empfangseinheit (3) und
- einem in den Patientenkörper implantierbaren telemetrischen Rejektionssensor (6), umfassend
   -- eine Hochfrequenz-Empfangseinheit (9') zum Empfang von Hochfrequenzsignalen von der Basisstation,
   -- eine Gleichrichter- und Dekodier-Einheit (18) zum Umsetzen der empfangenen Hochfrequenzsignale in eine Versorgungsspannung und Steueranweisungen für den Rejektionssensor(6),
   -- einen Energiespeicher (19) zum Speichern der Versorgungsspannung,
   -- eine Kontrolleinheit (20) zum Steuern der vorrichtungsinternen Meßvorgänge,
   -- eine mit dem zu überwachenden Organ in Kontakt bringbare, von Kontrolleinheit (20) gesteuerte Sensoranordnung (12) mit Meßelektroden (13, 14, 15, 16) zur Messung rejektionsspezifischer Größen,
   -- eine Kodiereinheit (29) zum Umsetzen der ausgewerteten Meßdaten in entsprechende Datensignale, sowie
   -- eine Hochfrequenz-Sendeeinheit (31) zum Senden der von der Kodiereinheit (29) erzeugten Datensignale zu der extrakorporalen Basisstation (1).

## Beschreibung

Die Erfindung betrifft eine Vorrichtung zur Rejektionsdiagnostik nach Organtransplantationen.

Zum Hintergrund der vorliegenden Erfindung ist auszuführen, daß ein Hauptproblem nach erfolgter Transplantation eine mögliche Abstoßung des transplantierten Organs aufgrund einer entsprechenden Abwehrreaktion des Patientenkörpers ist. Um dies zu unterbinden, wird eine medikamentöse Immunsuppression durchgeführt, die die Abstoßungsreaktionen unterdrucken soll.

Die Nebeneffekte und -wirkungen dieser medikamentösen Immunsuppression sind erheblich, so daß die Nachsorge des Patienten stets in dem Spannungsfeld von zu hoher Immunsuppression - mit den eventuell letalen Folgen einer Infektion - und einer zu geringen Immunsuppression - mit den eventuell letalen Folgen einer akuten Abstoßungsreaktion - steht. Aus diesem Grunde wird versucht, mit einer minimalen Basistherapie auszukommen und eventuell auftretende Abstoßungsepisoden frühzeitig zu erkennen. Diese können dann noch durch eine kurzfristig erhöhte Medikamentendosis abgefangen werden. Um diese Art von Therapie realisieren zu können, muß eine Methode zur Verfügung stehen, die eine rasche Erkennung einer Abstoßungsreaktion zuläßt. In diesem Zusammenhang ist bis heute die Biopsie üblich, bei der mikroinvasiv Gewebe aus dem transplantierten Organ entnommen und anschließend einer histologischen Untersuchung unterzogen wird. Aus dem Ergebnis dieser histologischen Untersuchungen können Rückschlüsse über eventuell zu erwartende Abstoßungsreaktionen gezogen werden.

Nachteilig bei einer solchen Biopsie-Überwachung ist die Tatsache, daß diese sehr aufwendig und mit relativ hohen Kosten verbunden ist. So muß mit einem Entnahmerhythmus zwischen zwei Wochen und zwei Monaten gearbeitet werden, wobei jedesmal ein für den Patienten unangenehmer mikroinvasiver Eingriff notwendig ist.

Es bestehen bereits Lösungsansätze für die Problematik dahingehend, daß elektrische Reiz-Antwort-Signale des transplantierten Organs analysiert und daraus auf dessen Zustand hinsichtlich einer möglichen Abstoßungsreaktion geschlossen wird. Dieses Diagnoseverfahren ist jedoch bis heute nicht etabliert und nur bei transplantierten Herzen anwendbar, da nur Herzen überhaupt entsprechende Reiz-Antwort-Signale zeigen. Andere Transplantationsorgane sind folglich nicht überwachbar. Zudem ist weiterhin von Nachteil, daß ein komplettes Herzschrittmacherssystem mit EKG-Telemetrie implantiert werden muß, um dieses Diagnoseverfahren durchführen zu können. Solche Herzschrittmachersysteme sind aufgrund der darin integrierten Batterie relativ groß und weisen nur eine begrenzte Lebensdauer auf. Nach Erschöpfen der Batterie muß das Herzschrittmachersystem ausgetauscht werden, was es für den Langzeiteinsatz bei der Rejektionsdiagnostik nicht prädestiniert.

Ausgehend von den geschilderten Problemen beim Stand der Technik liegt der Erfindung die Aufgabe zugrunde, eine Vorrichtung zur Rejektionsdiagnostik nach Organ-Transplantationen zur Verfügung zu stellen, die einerseits implantierbar ist, um jederzeit Informationen über den Zustand des transplantierten Organs telemetrisch zu erhalten, die andererseits jedoch nicht den Beschränkungen der vorstehend erwähnten, kompletten Herzschrittmacher-Systeme unterliegt.

Die Lösung dieser Aufgabe ist durch die im Anspruch 1 angegebenen Merkmale gelöst. Demnach ist eine Vorrichtung zur Rejektionsdiagnostik nach Organ-Transplantationen vorgesehen, die mit einer extrakorporalen Basisstation mit Hochfrequenz-Sende- und -empfangseinheit versehen ist. Ferner ist ein in den Patientenkörper implantierbarer telemetrischer Rejektionssensor vorgesehen, der
- eine Hochfrequenz-Empfangseinheit zum Empfang von Hochfrequenzsignalen von der Basisstation,
- eine Gleichrichter- und Dekodiereinheit zum Umsetzen der empfangenen Hochfrequenzsignale in eine Versorgungsspannung und Steueranweisungen für den Rejektionssensor,
- einen Energiespeicher zum Speichern der Versorgungsspannung,
- eine Kontrolleinheit zum Steuern der vorrichtungsinternen Meßvorgänge,
- eine mit dem zu überwachenden Organ in Kontakt bringbare, von der Kontrolleinheit gesteuerte Sensoranordnung mit Meßelektroden zur Messung rejektionsspezifischer Meßgrößen,
- eine Kodiereinheit zum Umsetzen der von der Sensoranordnung gelieferten Meßdaten in entsprechende Datensignale, und
- eine Hochfrequenz-Sendeeinheit zum Senden der von der Kodiereinheit erzeugten Datensignale zu der extrakorporalen Basisstation
aufweist.

Wie aus der vorstehenden Merkmalsauflistung deutlich wird, weist die erfindungsgemäße Vorrichtung keine Batterie mehr auf, wie sie bei aktiven Implantaten nach dem Stand der Technik notwendig ist. Insofern unterliegt die Vorrichtung keiner Lebensdauerbeschränkung durch die Energieversorgung. Bei der erfindungsgemäßen Vorrichtung handelt es sich vielmehr um ein passives, d.h. batterieloses System, bei der die notwendige Energie zur Stromversorgung der einzelnen Komponenten von außen eingekoppelt wird. Eine solche Einkopplung ist grundsätzlich bereits aus dem Stand der Technik in verschiedensten Bereichen als sogenanntes 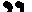 Transponder-System" bekannt. So werden Transponder-Systeme zur Identifizierung von Gegenständen oder zum Diebstahlsschutz bei Kraftfahrzeugen eingesetzt. Aus der WO94/04094 ist es beispielsweise bekannt, Prothesen, wie z. B. Brustimplantate mit einem kodierten Transponder zu versehen, der über ein Lesegerät von außen abzufragen ist, um z. B. Transplantationsdaten und Herstellerangaben auslesen zu können.

Grundsätzlich wird bei solchen Transponder-Systemen eine hochfrequente Trägerfrequenz in der Größenordnung ab einigen 100 kHz aufwärts von außen eingekoppelt und - je nach Systemauslegung - in der Regel zum Aufladen eines Versorgungskondensators genutzt, der wiederum einen Sender zum Rücksenden der im Transponder gespeicherten Daten mit Energie versorgt.

Im Rahmen der Erfindung wurde nun erkannt, daß durch eine Integration einer Meßvorrichtung für rejektionsspezifische elektrische Größen in ein implantierbares Transponder-System eine zuverlässige und dauerhafte Überwachung des jeweiligen Transplantates auf bevorstehende Abstoßungsreaktionen möglich ist. Der Rejektionssensor kann dabei so ausgelegt sein, daß verschiedene Meßaufgaben, wie die Messung autonomer elektrischer Größen (z.B.intrakardiales EKG), die Messung elektrischer Reiz-Antworten (sogenannter 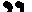 evozierter elektrischer Größen"), die Messung der Gewebeimpedanz und die Messung der Reizleitungsgeschwindigkeit im überwachten Transplantat kombiniert werden. Werden mehrere dieser Meßmethoden miteinander verknüpft, ist der Rejektionssensor als Multiplex-System auszulegen, das von außen programmierbar ist. Der Rejektionssensor kann jedoch auch von vornherein für eine gewünschte Meßaufgabe konfiguriert sein.

Zu bevorzugten Ausführungsformen der Diagnostikvorrichtung ist ferner festzuhalten, daß der gesamte Rejektionssensor einerseits in ein Gußgehäuse aus Silikonmaterial gekapselt sein kann, bei dem die Meßelektroden an der Oberfläche zu Tage treten. Dabei können die elektronischen Bauelemente des Sensors einschließlich der Meßelektroden auf einem IC-Baustein integriert sein. Alternativ dazu kann der eigentliche Elektronikteil des Rejektionssensors als gekapseltes Bauteil unter die Haut des Patienten eingepflanzt sein, wobei die Meßelektroden als gesonderte, an das zu überwachende Transplantat anlegbare Einheit ausgestaltet sind. Hierdurch werden die Telemetrieverluste bei der Datenübertragung zwischen dem extrakorporalen Basisgerät und dem Rejektionssensor minimiert. Trotzdem kann mittels der über Zuleitungen mit dem Rejektionssensor gekoppelten Meßelektrodeneinheit jedwedes Transplantat im Körper überwacht werden.

Bezüglich der extrakorporalen Basisstation ist festzuhalten, daß diese vorteilhafterweise in ein einfaches Empfangs- und Weitergabegerät und ein intelligentes Datenerfassungssystem aufgeteilt sein kann. Ein Beispiel dafür ist das sogenannte Telefon-Monitoring, wobei ein einfaches Empfangs- und Weitergabegerät als extrakorporale Basisstation im Besitz des Patienten ist und die Meßwerterfassung des Rejektionssensors steuert. Die von diesem empfangenen Daten werden über Telefon an ein zentrales Datenerfassungs- und -auswertungssystem - also z. B. an einen in einer Klinik stationierten, zentralen Auswerterechner - weitergeleitet.

Bevorzugte Ausführungsformen der erfindungsgemäßen Vorrichtung sowie weitere Merkmale, Einzelheiten und Vorteile davon sind den Unteransprüchen und der folgenden Beschreibung entnehmbar, in der ein Ausführungsbeispiel des Erfindungsgegenstandes anhand der beigefügten Zeichnungen näher erläutert wird. Es zeigen:
- Fig. 1: eine schematische Darstellung eines Rejektionssensors mit zugeordneter Basisstation und
- Fig. 2: ein Blockdiagramm der Komponenten des Rejektionssensors.

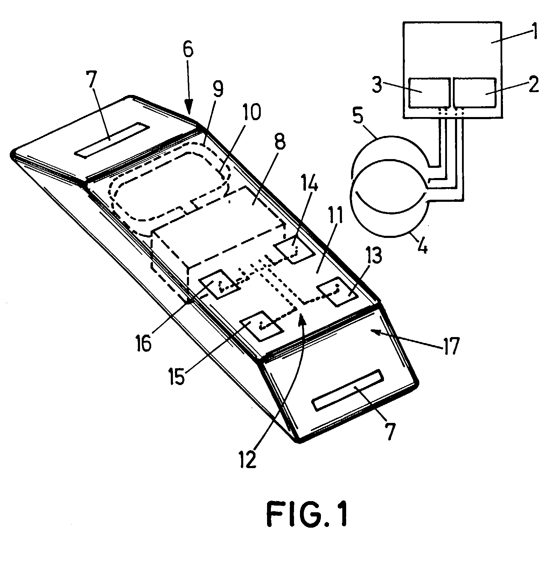

Aus der höchst schematischen Fig. 1 werden die zwei Hauptkomponenten der erfindungsgemäßen Vorrichtung zur Rejektionsdiagnostik deutlich. Es handelt sich zum einen um eine extrakorporale Basisstation 1, die eine Hochfrequenz-Sendeeinheit 2 und eine Hochfrequenz-Empfangseinheit 3 aufweist. Der Hochfrequenz-Sende- bzw. Empfangseinheit 2,3 sind jeweils eine Sendeantenne 4 bzw. eine Empfangsantenne 5 zugeordnet, über die amplitudenmodulierte (oder auch frequenzmodulierte) Hochfrequenzsignale zwischen der Basisstation 1 und dem implantierbaren Rejektionssensor 6 übertragbar sind. Die Antennen können auch als sogenannte Antennen-Arrays ausgelegt sein.

Der Rejektionssensor 6, der in Fig. 1 höchst schematisch und stark vergrößert dargestellt ist, wird bei einer Organtransplantation auf dem Spenderorgan durch übliche chirurgische Befestigungselemente 7 (z. B. chirurgisches Nahtmaterial) so festgelegt, daß an der Oberfläche des Organs in noch näher zu erläuternder Weise elektrische und elektrochemische Messungen durchgeführt werden können, die Aufschluß über eine möglicherweise bevorstehende Abstoßungsreaktion des Transplantates ergeben. Auch optische Messungen, Wärmeleitfähigkeitsmessungen oder ganz allgemein Temperaturmessungen können - je nach Anwendungsfall - mit dem Rejektionssensor durchgeführt werden.

Wie in Fig. 1 angedeutet ist, sind die elektronischen Komponenten des implantierbaren, telemetrischen Rejektionssensors 6 auf einem IC-Baustein 8 zusammengefaßt. Diesem sind wiederum eine Empfangsspule 9 und eine Sendespule 10 zugeordnet, über die die erwähnten Hochfrequenzsignale von der Basisstation empfangen bzw. dorthin gesendet werden können. Ferner ist an einer der ebenen Oberflächen 11 des Rejektionssensors 6 eine Sensoranordnung 12 mit vier miniaturisierten Meßelektroden 13, 14, 15, 16 vorgesehen, die an der Oberfläche 11 zutagetreten. Es können dabei die Meßelektroden 13, 14, 15, 16 und Spulen 9, 10 auch mit in den IC-Baustein 8 integriert sein.

Im übrigen sind die Sensoranordnung 12, die Empfangs- und Sendespulen 9, 10 und der Chip 8 in ein Gußgehäuse 17 aus Silikonmaterial gekapselt. Auch der Einbau in ein Titangehäuse ist denkbar.

Alternativ dazu können - wie bildlich nicht näher dargestellt ist - Sensor und Elektrodenanordnung getrennt sein, so daß der Rejektionssensor nicht auf dem Transplantat verankert sein muß. Es genügt, die Elektrodenanordnung, bei der es sich um ein sogenanntes 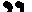 Mikroelektroden-Array" handeln kann, am transplantierten Organ festzulegen, wie dies von der Herzschrittmacher-Technik her bekannt ist.

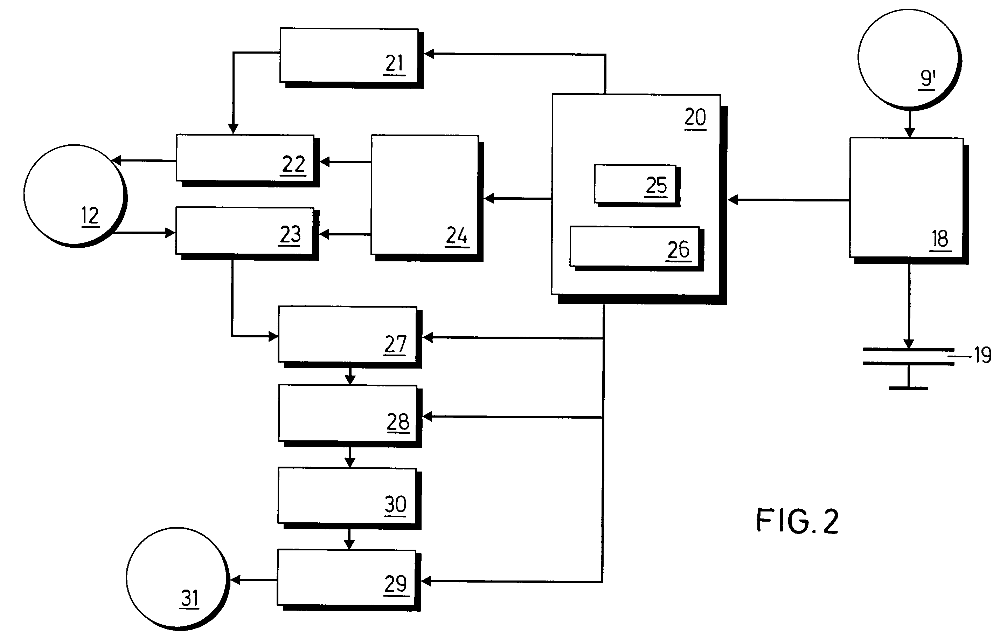

Anhand von Fig. 2 ist der Aufbau des eigentlichen Rejektionssensors 6 zu erläutern. So ist der die Empfangsspule 9 umfassenden Empfangseinheit 9' eine Gleichrichter- und Dekodiereinheit 18 nachgeschaltet, die aus dem empfangenen amplitudenmodulierten Hochfrequenzsignal die Steuerungsdaten für den Rejektionssensor 6 herausfiltert und mit der durch das Hochfrequenzsignal induzierten Spannung einen Versorgungskondensator 19 speist. Letzterer dient der Spannungsversorgung für alle elektronischen Komponenten des Rejektionssensors 6, was der Übersichtlichkeit halber jedoch in Fig. 2 nicht dargestellt ist. Im übrigen ist der Versorgungskondensators 19 in nicht näher dargestellter Weise mit einer Spannungsstabilisation versehen.

Die Gleichrichter- und Dekodiereinheit 18 ist durch ein nicht näher dargestelltes Netzwerk mit Demodulationswirkung realisiert, das die Steuerungsdaten aus dem HF-Signal extrahiert. Die übertragenen Steuersignale dienen dabei zur Auswahl des Meßverfahrens und zur Übermittlung der dabei zu verwendenden Meßparameter. Insofern werden die Steuersignale einer der Gleichrichter- und Dekodiereinheit nachgeschalteten Kontrolleinheit 20 zugeführt, die eine Signalgenerierungseinheit 21 sowie flexibel beschaltbare D/A-Multiplexer bzw. A/D-Multiplexer 22, 23 über eine entsprechende Multiplexer-Steuerlogik 24 steuert. Die Kontrolleinheit 20 weist ferner einen geeigneten Zeitgeber 25 auf, um zeitrelevante Meßverfahren, wie die Messung der Reizleitungsgeschwindigkeit in dem Organgewebe, ausführen zu können. Es ist außerdem eine Triggereinheit 26 in der Kontrolleinheit 20 vorgesehen, die eine dem A/D-Multiplexer 23 nachgeschaltete Signalkonditionierungseinheit 27, einen A/D-Wandler 28 und einen Datenkonditionierungseinheit 29 triggert. Die Signalkonditionierungseinheit 27 mit A/D-Wandler 28 stellt eine Auswerteeinheit für die Meßsignale dar. Zwischen A/D-Wandler 28 und Datenkonditionierungseinheit 29 ist ein Speicher 30 z. B. in Form eines FIFO-Speichers zwischengeschaltet. Der Datenkonditionierungseinheit 29, die eine Kodierung der ausgewerteten Meßdaten in entsprechende Datensignale vornimmt, ist eine HF-Ausgabeeinheit 31 nachgeschaltet, die eine Datensignalübertragung auf die Sendespule 10 zur Basisstation 1 hin veranlaßt.

Soll nun der Rejektionssensor 6 das von ihm überwachte Transplantat beispielsweise durch Messung der Reizleitungsgeschwindigkeit im Organgewebe überwachen, wird ein entsprechend kodiertes HF-Signal von der Basisstation 1 über die HF-Sendeeinheit 2 zum Rejektionssensor 6 telemetrisch übermittelt. Die Gleichrichter- und Dekodiereinheit 18 extrahiert aus diesem Signal die Steuerungsdaten. Gleichzeitig wird der Versorgungskondensator 19 (weiter) aufgeladen.

Die Kontrolleinheit 20 übernimmt die aus dem HF-Signal extrahierten Daten und dekodiert diese zur Auswahl des gewünschten Meßverfahrens, im vorliegenden Falle also der Messung der Reizleitungsgeschwindigkeit. Daraus wird entsprechend über die Signalgenerierungseinheit 21 die multiplexe Steuerlogik 24 angesteuert. Erstere paßt die an die Meßelektroden 13 bis 16 der Sensoranordnung 12 für das entsprechende Meßverfahren auszugebenden Signale der Meßaufgabe an. In der Signalgenerierungseinheit 20 kann also eine Wechselspannung, z. B. für die Messung der Gewebeimpedanz erzeugt werden oder ein Generator für Konstantspannungsstöße implementiert sein, um elektrische Reiz-Antworten zu messen. Die Multiplexer-Steuerlogik 24 steuert die einzelnen die Verbindung zu den Meßelektroden 13 bis 16 herstellenden Multiplexer 22, 23 für die Ein- und Ausgabe von Meßsignalen.

Die von den Meßelektroden 13 bis 16 der Sensoranordnung 12 zum A/D-Multiplexer 23 herangeführten Signale werden durch die Signalkonditionierungseinheit 27 ausgewertet, also z. B. einer Filterung oder Mittelwertbildung unterzogen. Der nachfolgende A/D-Wandler 28 setzt die Meßsignale in digitale Form um, um sie in dem Speicher 30 zwischenzuspeichern. Die Datenkonditioniereinheit 29 kodiert und komprimiert die Meßdaten zur Sicherung gegenüber Übertragungsfehlern und gibt die so bearbeiteten Daten zur HF-Ausgabeeinheit 31 weiter, um entsprechende Datensignale über die Sendespule 10 der Basisstation 1 zu übermitteln. Dort werden die Meßdaten empfangen, umgesetzt und entsprechend zur Anzeige gebracht.

Durch die Trennung der HF-Empfangs- 9' und HF-Ausgabeeinheit 31 können Energieversorgung und Steuerung einerseits und die Datenübertragung andererseits zweikanalig auf unterschiedlichen Frequenzen und mit unterschiedlichen Übertragungsraten gleichzeitig erfolgen.

Durch den erläuterten Aufbau kann der Rejektionssensor unter Steuerung von außen verschiedene Meßverfahren durchführen. So können autonome elektrische Größen, wie z. B. ein EKG gemessen werden. Denkbar sind auch chemische Sensoren zur Erfassung bestimmter chemischer Zusammensetzungen oder Anteile auf dem Transplantat. Bei der Messung elektrischer Reiz-Antworten, werden über zwei Meßelektroden 13, 14 ein Spannungsimpuls in das Gewebe eingebracht und über die beiden anderen Meßelektroden 15, 16 ein entsprechende Reiz-Antwort des Organs erfaßt.

Bei der Messung der Gewebs-Impedanz wird über zwei Meßelektroden 13, 15 ein Wechselstrom mit veränderlicher Frequenz eingeprägt und die über die Elektroden abfallende Spannung gemessen. Damit wird ein komplettes Impedanz-Spektrum des überwachten Transplantates erstellt.

Zur Messung der Reizleitungsgeschwindigkeit wird über zwei Meßelektroden 13, 14 ein Spannungsimpuls eingebracht und die Zeit gemessen, bis dieser Reiz an dem zweiten Elektrodenpaar 15, 16 angekommen ist. Damit kann auf die Reizleitungsgeschwindigkeit rückgerechnet werden.

In einer etwas vereinfachten Ausführungsform des Erfindungsgegenstandes, wie sie nicht eigens dargestellt ist, können einerseits die Sende- und Empfangsspulen 31, 9' zu einer einzigen Spule und andererseits die beiden D/A bzw. A/D Multiplexer zu einem einfachen Multiplexer zusammengefaßt werden. Bei Verwendung einer einzigen Antenne können durch Einsatz zweier Bandpaßfilter trotzdem Senden und Empfangen auf zwei unterschiedlichen Frequenzen stattfinden. Auch ist es möglich, auf den Speicher 30 zu verzichten, wenn die Meßdaten mittels Telemetrie bei ausreichend hoher Übertragungsgeschwindigkeit 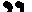 on-line" zur Basis-Station übertragen werden.

Die Multiplexer-Programmierung des erläuterten Rejektionssensors ist von Vorteil, um die verschiedenen Meßverfahren von außen abrufen zu können. Damit ist der Rejektionssensor universell einsetzbar und wechselnden Verhältnissen anpaßbar.

Falls eine entsprechende medizinische Indikation vorliegt, kann der Rejektionssenor auch nur für eine bestimmte Anwendung konfiguriert sein, wodurch sich der technische Aufwand natürlich reduziert.

## Patentansprüche

1. Vorrichtung zur Rejektionsdiagnostik nach Organ-Transplantationen mit
- einer extrakorporalen Basisstation (1) mit Hochfrequenz-Sende- (2) und Empfangseinheit (3) und
- einem in den Patienterkörper implantierbaren telemetrischen Rejektionssensor (6), umfassend
-- eine Hochfrequenz-Empfangseinheit (9') zum Empfang von Hochfrequenzsignalen von der Basisstation,
-- eine Gleichrichter- und Dekodier-Einheit (18) zum Umsetzen der empfangenen Hochfrequenzsignale in eine Versorgungsspannung und Steueranweisungen für den Rejektionssensor(6),
-- einen Energiespeicher (19) zum Speichern der Versorgungsspannung,
-- eine Kontrolleinheit (20) zum Steuern der vorrichtungsinternen Meßvorgänge,
-- eine mit dem zu überwachenden Organ in Kontakt bringbare, von der Kontrolleinheit (20) gesteuerte Sensoranordnung (12) mit Meßelektroden (13, 14, 15, 16) zur Messung rejektionsspezifischer Meßgrößen,
-- eine Kodiereinheit (29) zum Umsetzen der von der Sensoranordnung (12) gelieferten Meßdaten in entsprechende Datensignale, sowie
-- eine Hochfrequenz-Sendeeinheit (31) zum Senden der von der Kodiereinheit (29) erzeugten Datensignale zu der extrakorporalen Basisstation (1).

2. Diagnostikvorrichtung nach Anspruch 1, dadurch gekennzeichnet, daß der Energiespeicher ein der Gleichrichter- und Dekodiereinheit (18) nachgeschalteter Versorgungskondensator (19) ist.

3. Diagnostikvorrichtung nach Anspruch 1 oder 2, dadurch gekennzeichnet, daß die Hochfrequenz-Empfangseinheit (9') zum Empfang Amplituden- oder Frequenzmodulierter Hochfrequenzsignale ausgelegt ist.

4. Diagnostikvorrichtung nach einem der Ansprüche 1 bis 3, dadurch gekennzeichnet, daß die Sensoranordnung (12) als Multiplex-System ausgebildet ist.

5. Diagnostikvorrichtung nach Anspruch 4, dadurch gekennzeichnet, daß ein D/A- (22) und A/D-Multiplexer (23) und eine Multiplexer-Steuerlogik (24) zwischen der Sensoranordnung (12) und der Kontrolleinheit (20) zwischengeschaltet sind.

6. Diagnostikvorrichtung nach einem der Ansprüche 1 bis 5, dadurch gekennzeichnet, daß die Sensoranordnung (12) vier vorzugsweise im Rechteck angeordnete Meßelektroden (13, 14, 15, 16) aufweist, die unter Steuerung durch die Kontrolleinheit (20) für unterschiedliche Meßzwecke variabel zusammenschaltbar sind.

7. Diagnostikvorrichtung nach einem der Ansprüche 1 bis 6, dadurch gekennzeichnet, daß mittels der Sensoranordnung (12) Organ-spezifische elektrochemische Größen, die Gewebeimpedanz des Organs, elektrische Reiz-Antworten des Gewebes und/oder die Reizleitungsgeschwindigkeit des Gewebes als rejektionsrelevante Größen meßbar sind.

8. Diagnostikvorrichtung nach einem der Ansprüche 1 bis 7, dadurch gekennzeichnet, daß die Bauelemente des Rejektionssensors (6) in ein Gußgehäuse (17) aus Silikonmaterial gekapselt sind, bei dem die Meßelektroden (13, 14, 15, 16) an der Oberfläche (11) zutagetreten.

9. Diagnostikvorrichtung nach einem der Ansprüche 1 bis 8, dadurch gekennzeichnet, daß die Bauelemente des Rejektionssensors (6) einschließlich der Meßelektroden (13, 14, 15, 16) auf einem IC-Baustein (8) integriert sind.

10. Diagnostikvorrichtung nach einem der Ansprüche 1 bis 7, dadurch gekennzeichnet, daß einerseits der Rejektionssensor als gekapseltes, unter die Haut des Patienten einpflanzbares Bauteil und andererseits die Meßelektroden als gesonderte, an das zu überwachende Organ anlegbare Einheit ausgestaltet sind.

11. Diagnostikvorrichtung nach einem der Ansprüche 1 bis 10, dadurch gekennzeichnet, daß die Hochfrequenz-Empfangs- (9') und -Sende-Einheit (31) getrennt sind und mit unterschiedlichen Frequenzen arbeiten.

12. Diagnostikvorrichtung nach einem der Ansprüche 1 bis 11, dadurch gekennzeichnet, daß der Reaktionssensor (6) Befestigungselemente (7) zur Fixierung an der Oberfläche (11) des zu überwachenden Transplantates aufweist.

13. Diagnostikvorrichtung nach einem der Ansprüche 1 bis 12, dadurch gekennzeichnet, daß die extrakorporale Basisstation in ein dem Patienten zugeordnetes Datenempfangs- und -weitergabegerät einerseits und ein zentrales Datenerfassung- und -auswertungssystem andererseits geteilt ist.
